# EUROPEAN PATENT SPECIFICATION

(11) **EP 4 420 615 B1**
(45) Date of publication and mention of the grant of the patent: **06.05.2026**
(21) Application number: 21961123.3
(22) Date of filing: 27.10.2021
(51) Int. Cl.: A61N 5/06, A61B 10/00

(54) **TRANSCRANIAL LIGHT REGULATION APPARATUS**
TRANSKRANIELLE LICHTREGULIERUNGSVORRICHTUNG
APPAREIL DE RÉGULATION DE LUMIÈRE TRANSCRÂNIENNE

(30) Priority: 19.10.2021 CN 202111217554
(43) Date of publication of application: 28.08.2024
(73) Proprietor: Danyang Huichuang Medical Equipment Co., Ltd., Zhenjiang, Jiangsu 212300 (CN)
(72) Inventor: WANG, Daifa, Beijing 100080 (CN)
(74) Representative: Ziebig Hengelhaupt Intellectual Property Attorneys Patentanwaltskanzlei PartGmbB
(86) International application number: PCT/CN2021/126700
(87) International publication number: WO 2023/065381

(56) References cited:
- WO-A1-2021/068278
- CN-A- 102 811 668
- CN-A- 102 811 668
- CN-A- 107 106 063
- CN-A- 111 839 531
- CN-A- 111 973 886
- JP-A- 2018 068 595
- KR-B1- 101 273 613
- US-A1- 2013 310 676
- US-A1- 2013 310 676
- US-A1- 2016 235 983
- US-A1- 2018 015 301

## Description

### TECHNICAL FIELD

The present disclosure relates to a technical field of transcranial light regulation method and equipment, especially relates to a hair parting assembly, also said light guide device for transcranial light, and an apparatus for transcranial light regulation.

### BACKGROUND

Research has shown that red light or near-infrared light with a certain wavelength can penetrate the human skull and produce beneficial effects on cells by stimulating them, thereby achieving the goal of treating cerebral diseases, such as neurodegenerative diseases, psychiatric disorders, traumatic disorders, etc.

In the existing transcranial light regulation products, the optical assembly and the hair parting assembly are correspondingly provided and fixedly assembled together, it mainly provides corresponding light guide pillars on the optical assembly to interpose between the hairs, or part the hairs ("part the hairs" means to poke into and push the hairs aside in disclosure) by a drive assembly driving the correspondingly provided hair parting member from a gathered state to a deployed state, thereby achieving light guide effect (also referred to as a light guide assembly or a light guide device for transcranial light). It is difficult for the former to directly contact the root of the hair to transmit the transcranial light to the scalp, while the latter can only partially part the hair at the corresponding position of the optical assembly, degrading the transmission effect of the transcranial light, which in turn degrades the treatment effect of the transcranial light. Besides, the above two hair parting methods both depend on complicated mechanical structure and have high cost.

WO2021/068278A1 disclosed a light-emitting device, apparatus and system for transcranial light modulation. The device comprises: a light source portion; a light-transmitting layer, with the light-transmitting layer being arranged on an emergent side of the light source portion; and a light guide member, with the light guide member having one end fixed to the side of the light-transmitting layer that is opposite the light source portion, and being configured to guide light that is transmitted from the light-transmitting layer. By means of providing the light guide member on the light-emitting device for transcranial light modulation, the light guide member passes between the hair of a user and comes into direct contact with the scalp of the user so as to reduce the influence of hair shading on the light transmission efficiency, and the light emitted by the light source portion can be directly guided to the scalp of the user by means of the light guide member, so as to improve the irradiation effect of the light-emitting device. However, the light source portion and the light guide member are correspondingly provided and fixed assembled together, and corresponding light guide pillars are provided on the light source portion to interpose between the hairs, which makes it difficult to directly contact the root of the hair to transmit the transcranial light to the scalp.

### SUMMARY

The invention relates to a device as defined in the appended claims. Embodiments, examples or aspects in the following disclosure which do not fall under the scope of the claims are presented for illustration purposes only and do not form part of the invention.

In response to the aforementioned technical issues in the prior art, the present invention, which is identified in the appended set of claims, provides a hair parting assembly (also referred to as a light guide device for transcranial light) and a transcranial light regulation apparatus, which can part and push the hair on the user's scalp aside by means of the hair-entering member to form an optical path, thereby transmitting transcranial light to the scalp. By means of the hair parting assembly, a hair parting device, and a transcranial light regulation apparatus , the light transmission rate is high, the structure is simple, and the cost is lower.

According to the first aspect of the present disclosure, a hair parting assembly is provided as in claim 1. It comprises a first bridging portion and at least one first hair-entering member for transmitting transcranial light. The first bridging portion at least partially extends in a circumferential direction in a strip-shape or a band-shape. Each first hair-entering member includes a hair-entering portion and a maintaining portion, both of which are permeable to the transcranial light and is provided traverse to the first bridging portion, such that a lengthwise direction of each first hair-entering member is traverse to extension direction of the first bridging portion, and spaces are provided on both sides of each first hair-entering member in the extension direction of the first bridging portion. The first bridging portion is configured to travel along the scalp of a user, the first hair-entering member is configured to part the hairs as the first bridging portion travels to expose the scalp in the traveling path to form an optical path, and the maintaining portion is configured to maintain the optical path via which external transcranial light is transmitted to the scalp.

According to a further aspect of the present disclosure, a transcranial light regulation apparatus is provided, which comprises a carrying member and an optical assembly provided on the carrying member for transmitting transcranial light. It further comprises at least one light guide device according to the present invention.

Compared with the prior art, the beneficial effect of the embodiments of the present disclosure is that the present disclosure parts the hairs on the scalp of a user by means of the hair-entering member provided to the bridging portion to form an optical path, so that the transcranial light is transmitted to the scalp, and the light transmission rate is high. The bridging portion enables the hair parting assembly to be worn stably on the head of the user, and adapt to head forms and sizes of different users. Moreover, the abovementioned structure provided by present disclosure is not limited by factors such as the structure and the arrangement position of an external optical assembly, the structure is simple meanwhile the cost is low.

### BRIEF DESCRIPTION OF THE DRAWINGS

In figures that are not necessarily drawn to scale, the same reference numerals may describe similar components in different figures. The same reference signs with suffixes or different suffixes may denote different examples of similar components. The figures generally show various embodiments by way of example rather than limitation, and are used together with the description and the claims to describe the embodiments of the present disclosure. As proper, the same reference sign may be used throughout the drawings to denote the same or similar part. Such embodiments are illustrative, and are not intended to be exhaustive or exclusive embodiments of the present device or method.
FIG.1 is a first structural schematic diagram of the hair parting assembly according to the embodiment of the present disclosure;
FIG.2 is a second structural schematic diagram of the hair parting assembly according to the embodiment of the present disclosure;
FIG. 3 is a third structural schematic diagram of the hair parting assembly according to the embodiment of the present disclosure;
FIG. 4 is a fourth structural schematic diagram of the hair parting assembly according to the embodiment of the present disclosure;
FIG. 5 is a fifth structural schematic diagram of the hair parting assembly according to the embodiment of the present disclosure;
FIG. 6 is a sixth structural schematic diagram of the hair parting assembly according to the embodiment of the present disclosure;
FIG. 7 is a structural schematic diagram of the hair parting device in an operation state according to the embodiment of the present disclosure;
FIG. 8 is a structural schematic diagram of the hair parting assembly according to another embodiment of the present disclosure;
FIG. 9 is a structural schematic diagram of the transcranial light regulation apparatus according to the embodiment of the present disclosure.

The components indicated by the reference signs in the figures are as follows:
100- hair parting assembly; 110- first bridging portion; 120- first hair-entering member; 121- hair-entering portion; 122- maintaining portion; 130- first fixing member; 131-mounting hole; 200- hair parting device; 201- rotating shaft; 210- second fixing member; 101- first hair parting assembly; 102- second hair parting assembly; 300- hair parting assembly; 310- second bridging portion; 320- second hair-entering member; 311- base; 312- grasping portion; 313- light guide member; 322- comb teeth portion; 400-transcranial light regulation apparatus; 410- carrying member; 420- optical assembly; 430- hair parting assembly.

### DETAILED DESCRIPTION

In order to enable those skilled in the art to better understand the technical solutions of the present disclosure, the present disclosure will be described in detail below in conjunction with the accompanying drawings and detailed embodiments. Embodiments of the present disclosure will be described in further detail below in conjunction with the accompanying drawings and detailed embodiments, but this is not intended to limit the present disclosure.

"First", "second" and similar words used in the present disclosure do not indicate any sequence, quantity or importance, but are only used for distincting different parts. Words like "including" or "comprising" mean that the elements preceding the word cover the elements listed after the word, and do not exclude the possibility of also covering other elements. The terms "up", "down", "left", "right", etc. are only used to represent relative positional relationships. When the absolute position of the described object changes, the relative positional relationship may also change accordingly.

In the present disclosure, when describing a specific device located between a first device and a second device, there may or may not be an intermediate device between the specific device and the first or second device. When describing the connection of a specific device to other devices, the specific device can be connected directly to the other devices without an intermediate device, or it can be connected indirectly to the other devices with an intermediate device.

All terms used in the present disclosure (including technical terms or scientific terms) have the same meanings as those understood by those skilled in the art to which the present disclosure belongs, unless specifically defined. It should also be understood that terms defined in general dictionaries should be interpreted as having meanings that are consistent with their meanings in the context of the relevant technology, and should not be interpreted in idealized or overly formal terms, unless explicitly defined here.

The techniques, methods, and equipments known to those skilled in the relevant art may not be discussed in detail, but in appropriate cases, the techniques, methods, and equipments should be considered as a part of the description.

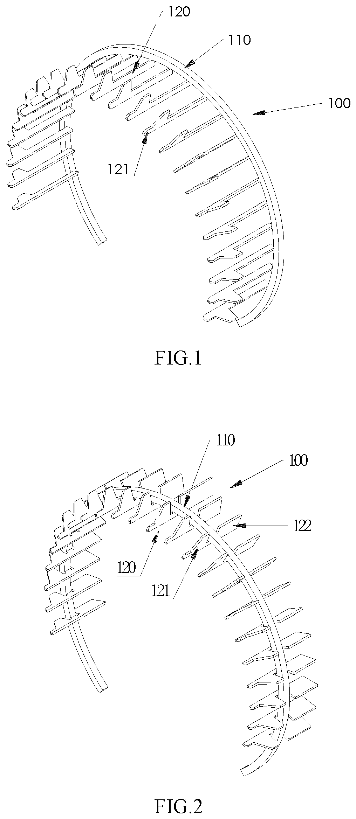

According to a first embodiment of the present disclosure, a hair parting assembly 100 is provided. The hair parting assembly herein is used for parting (poking into and pushing aside) and fixing the hairs in order to form an optical path to guide the transcranial light, which is also referred to as a light guide device for transcranial light. As shown in FIGS. 1-5, the hair parting assembly 100 comprises a first bridging portion 110 and at least one first hair-entering member 120 for transmitting transcranial light. The first bridging portion 110 is constructed to extend at least partially in a circumferential direction (the first bridging portion 110 may also be composed of a plurality of portions that do not have a connecting relationship, as long as the first bridging portion 110 has an extension at least partially in a circumferential direction) and to have elasticity, so as to enable the hair parting assembly 100 to be stably worn on a user's head and to adapt to head forms and sizes of different users. Each first hair-entering member 120 is provided to the first bridging portion 100, such that the lengthwise direction of each first hair-entering member 120 intersects the extension direction of the first bridging portion 110, and spaces are provided on both sides of each first hair-entering member 120 in the extension direction of the first bridging portion 110. That is, there are reserved intervals.

It should be noted that, the hair parting assembly 100 in the present disclosure serves as a separate light transmission assembly, which can transmit (guide) transcranial light emitted by an external optical assembly to the scalp of a user, that is, the hair parting assembly 100 can be used in cooperation with the external optical assembly, but is not provided on the same member.

Particularly, the first hair-entering member 120 can be interposed between the hairs of the user and contact the scalp, and the first bridging portion 110 bridges the respective first hair-entering members 120 at a certain angle (intersectively, e.g., in the horizontal direction or in the vertical direction), such that the respective first hair-entering members 120 distributed on the first bridging portion 110 form a comb teeth shape. When the first bridging portion 110 slides along the scalp, the first hair-entering members 120 move along the traveling direction of the first bridging portion 110, and in the process of traveling, the first hair-entering members 120 gradually part the hairs at the first hair-entering members 120 to expose the scalp position in the traveling path to form an optical path, so that external transcranial light can be transmitted to the scalp of the user through the optical path.

Further, spaces are provided on both sides of each first hair-entering member 120 in the extension direction of the first bridging portion 110. That is, there are reserved intervals. The spaces are used to converge and bunch the hairs parted by the first hair-entering member 120, in order to avoid the hair from being disheveled, and thus to avoid the formed optical path from being covered by the disheveled hairs and interfering with the optical path. Specifically, the size of the space may be determined according to the hair property of the user to achieve a better transmission effect of the transcranial light. As an example, the size of the space may be determined according to the sparseness of the hairs of the user, so that for a user with sparse hairs, a smaller space may be sufficient for converging and bunching the hairs parted by the first hair-entering member 120.

It will be appreciated that, depending on the target irradiation area of the transcranial light, the first hair-entering member 120 may be provided as one or more to facilitate the transmission of more transcranial light onto the target irradiation area when parting hairs using the hair parting assembly 100. In some embodiments, an intersection angle between the lengthwise direction of the first hair-entering member 120 and the extension direction of the first bridging portion 110 may be set according to the distribution position of the hairs on the scalp, head forms and sizes, the target irradiation position of the transcranial light, and the like, so as to achieve a better hair parting effect, transmit as much transcranial light as possible to the scalp of the user, and improve the light transmission rate. For example, the lengthwise direction of the first hair-entering member 120 on the first bridging portion 100 corresponding to the middle position of the user's head is set to be perpendicular to the extension direction of the first bridging portion 100, so that the first hair-entering member 120 can better part the hairs in the forehead and the top region, and thus can transmit more transcranial light to the scalp in the forehead and the top region.

It can be understood that the first hair-entering assembly 120 and the first bridging portion 110 may be of an integrated molding structure, or may be connected in a detachable manner, so as to make the hair parting assembly 100 more personalized, and the user may assemble the first hair-entering assembly 120 in position according to the actual needs. For example, when the target irradiation region of the transcranial light is the forehead, the first hair-entering assembly 120 may be assembled to a position of the first bridging portion 110 corresponding to the forehead region. For example, when the transcranial light irradiates the temporal region, the first hair-entering member 120 may be assembled to a position of the first bridging portion 110 corresponding to the temporal region. In another embodiment, the first hair-entering members 120 with different shapes and sizes can be assembled according to the hair property of the user, so as to achieve a better transmission effect of the transcranial light.

It will be appreciated that the first bridging portion 110 may be constructed as any structure that can adapt to the shape of the user's head, such as arc, semi arc, or ellipse, and the present disclosure does not specifically limit this. In some embodiments, the first bridging portion 110 may be constructed as an elastic structure, or made of elastic material, or utilizing a circumferential extending shape (e.g., an arch) thereof to provide the elasticity, as long as it possesses a certain degree of elasticity that enables the first bridging portion 110 to be stably worn on the user's head, and the present disclosure does not make any specific limitations in this regard either.

It can be understood that the first hair-entering member 120 may be at least partially (e.g., a maintaining portion 122) made of material with light transmission (including but not limited to transparent, translucent, etc.,) or constructed as a light transmittable structure. As an example, in one embodiment, the first hair-entering member 120 is made of an acrylic material to achieve good transparent effect. In another embodiment, the first hair-entering member 120 may be constructed as a transparent hollow structure, and, in yet another embodiment, an optical fiber may be provided with for transmitting transcranial light in the hollow structure, as long as the first hair-entering member 120 can be used for transmitting transcranial light, and the present disclosure does not specifically limit this.

It can be understood that the present disclosure does not specifically limit the structural shape, number, size, softness or hardness of the material and other parameters of the first hair-entering member 120, so long as the first hair-entering member 120 can be smoothly interposed between the hairs of the user and part the hairs. For example, the first hair-entering member 120 may be constructed in any shape such as a plate shape, comb teeth shape, arc shape, etc. As shown in FIG. 1, the first hair-entering member 120 is constructed in the plate shape, and a tip (i.e., constructed as a hair-entering portion) may be formed at an end of the plate-shaped structure away from the first bridging portion 110, so as to be able to be smoothly interposed into the user's hairs. Specifically, the number and size of the first hair-entering member 120 may be set according to the hair property of the user to achieve a better hair parting effect, and the hair property may include hair sparseness, hair thickness, and the like. Preferably, the first hair-entering member 120 may be made of a material with a certain degree of elasticity, so that the user's scalp will not be harmed while the hairs are smoothly parted.

The hair parting assembly 100 provided in the present disclosure parts the hairs on the user's scalp by means of the first hair-entering member 120 provided on the first bridging portion 110 and forms an optical path at the first hair-entering member 120 and in its traveling path, so as to realize the transmission of transcranial light to the scalp. The first bridging portion 110, which at least partially extends in a circumferential direction and has elasticity, enables the hair parting assembly 100 to be worn stably on the user's head, and adapt to the head forms and sizes of different users. The hair parting assembly 100 can better part the user's hairs and transmit as much transcranial light as possible to the scalp through the formed optical path, thus the light transmission rate is high, without being limited by the structure, arrangement position, etc., of the external optical assembly, the structure is simple and the cost is low.

It should be noted that the present disclosure provides a plurality of embodiments, particularly including at least the embodiments hereinafter, wherein various technical features in each embodiment may be combined with each other without conflict to form other technical solutions capable of realizing transmission of the transcranial light by means of the hair-entering member provided on the bridging portion.

As shown in FIGS. 1-2, the first bridging portion 110 is constructed as an arc-shaped structure capable of being worn on a user's head, a plurality of first hair-entering members 120 are disposed along a circumferential direction of the first bridging portion 110 and are disposed on a side of the first bridging portion 110, and each of the first hair-entering members 120 has a hair-entering portion 121 and a maintaining portion 122 (which extends from the hair-entering portion 121 to a portion connected to the first bridging portion 110, as shown in FIG. 1,), and when in use, the hair-entering portion 121 is interposed between the hairs of the user, by sliding the first bridging portion 110 along the scalp, the first hair-entering member 120 provided thereon moves following the traveling direction of the first bridging portion 110 to part the hairs at the first hair-entering member 120, exposing the scalp in its traveling path to form an optical path at this position, and the optical path can be maintained by the maintaining portion 122, and transcranial light emitted by the external optical assembly is transmitted to the scalp of the user via the optical path.

Further, as shown in FIGS. 1-2, the bottom of the first bridging portion 110 has a first predetermined height with respect to the bottom of the first hair-entering member 120, such that there is a certain space between the first bridging portion 110 and the scalp, thereby enabling the depressing and converging of the height of the hairs in the spaces on the two sides of the first hair-entering member 120 during the travel of the first hair-entering member 120 to part the hair, so as to achieve a better converging, bunching and combing effect on the hair, reduce the degree of disheveling of the hair, and avoid the disheveled parted hair from covering the first hair-entering member 120 and the optical path formed in its traveling path.

It will be appreciated that at least a part (e.g., but not limited to the top) of the first hair-entering member 120 has a second predetermined height, which may be higher than the first predetermined height. Particularly, the second predetermined height of the first hair-entering member 120 is set to go beyond the first predetermined height, such that the at least part (e.g., but not limited to, the top) of the first hair-entering member 120 is beyond the height of the depressed hairs, which allows at least part of the first hair-entering member 120 goes beyond the surface of the hairs, thereby realizing that more transcranial light is received, and then allowing for more transcranial light to be transmitted to the scalp. Further, the second predetermined height of the first hair-entering member 120 is set to go beyond the first predetermined height, which can adapt to the change in height of the hairs due to the hairs being fluffed up under its own elasticity due to the pushing of the first bridging portion 110, so that even the hairs with such a change in height are still able to be maintained as split bundles by the first hair-entering member 120, thereby avoiding obstructing the optical path.

In some embodiments, the second predetermined height (or its exceeding height over the first predetermined height) is set associated with the hair property of the user. Particularly, the hairs have a certain thickness, and the second predetermined height of the first hair-entering member 120 at least goes beyond the thickness of the hairs. That is, the top of the first hair-entering member 120 having the second predetermined height is able to penetrate out of the surface of the hairs to receive the external transcranial light, so that the transcranial light can be transmitted to the scalp via the first hair-entering portion 120. If the first hair-entering portion 120 does not have the second predetermined height, the hair-entering portion 121 thereof can easily be covered by the hairs after being interposed between the hairs, resulting in less transcranial light or no transcranial light being transmitted to the scalp. As shown in FIGS. 1-2, the first hair-entering member 120 is constructed in plate shape, the plate-shaped structure is capable of maintaining an optical path formed between the hairs and the scalp, through which the external transcranial light is transmitted to the scalp at the bottom of the first hair-entering member 120, so as to realize a higher light transmission rate. It needs to be noted that the first hair-entering member 120 may be constructed in any shape, as long as it has a second predetermined height capable of maintaining the optical path formed between the hairs and the scalp, and the present disclosure does not specifically limit this.

Further, an exceeding height of the second predetermined height of the first hair-entering member 120 over the first predetermined height is set associated with the hair property of the user, which comprises at least one of hair thickness, hair amount, and hair elasticity. The exceeding height of the second predetermined height over the first predetermined height is set associated with the hair property to increase the light transmission rate. For example, when the hairs of the user are thicker and has more hair amount, the exceeding height needs to be set to a higher value to ensure that the top of the first hair-entering member 120 is beyond at least the surface of the hairs as a reference, so that more transcranial light can be received. When the user's hairs have more elasticity, the converged hairs tend to return and get disheveled, for this case, it is also necessary to set the exceeding height to a higher value.

It is understood that the person skilled in the art may set the length of the first hair-entering member 120 according to actual needs. Since the first hair-entering member 120 is bound to form an optical path on its traveling path during its traveling process, and the structural design of the first hair-entering member 120 and the first bridging portion 110 can make the parted hairs well converged, so that the optical path will not be completely obstructed by the converged hairs. Therefore, even if the length of the first hair-entering member 120 is relatively short, the optical path formed in the traveling path of the first hair-entering member 120 is not maintained by the first hair-entering member 120, the exposed scalp still may not be covered by the hair or only a small part of the optical path may be covered, then, a part of the transcranial light may also be transmitted to the scalp through the optical path formed in the traveling path of the first hair-entering member 120.

In a preferred embodiment, as shown in FIG. 1, the length of the first hair-entering member 120 is relatively long, such that the optical path formed by the first hair-entering member 120 in its traveling path can each be maintained by the first hair-entering member 120, and since the second predetermined height of the first hair-entering member 120 is higher than the thickness of the hairs, it passes out of the surface of the hair and receives more transcranial light, resulting in a high light transmission rate.

In some embodiments, each first hair-entering member 120 may include a hair-entering portion 121 and a maintaining portion 122, wherein, as shown in FIG. 1 or FIG. 2, the hair-entering portion 121 and the maintaining portion 122 are constructed to be distributed on two opposite sides or the same side of the first bridging portion 110. As shown in FIG. 2, the first hair-entering member 120 comprises a hair-entering portion 121 and a maintaining portion 122, the hair-entering portion 121 and the maintaining portion 122 are constructed to be distributed on two opposite sides of the first bridging portion 110, wherein the hair-entering portion 121 is used to be interposed between the hairs of the user so that the first hair-entering member 120 can smoothly travel on a surface of the scalp and part the hair to form an optical path, and the maintaining portion 122 is used to maintain the optical path formed during traveling process of the hair-entering portion 121 to avoid the parted hair from returning or getting disheveled to cover the optical path, so as to achieve a higher light transmission rate.

It should be noted that the connection relationship between the hair-entering portion 121 and the maintaining portion 122 is not particularly limited in the present disclosure, and the two may be constructed as an integrated molded structure, may be detachably connected, or may not have a connection relationship with each one assembled together with the first bridging portion 110 respectively, as long as it is possible to realize that the optical path formed by the hair-entering portion 121 in the traveling path is maintained by using the maintaining portion 122.

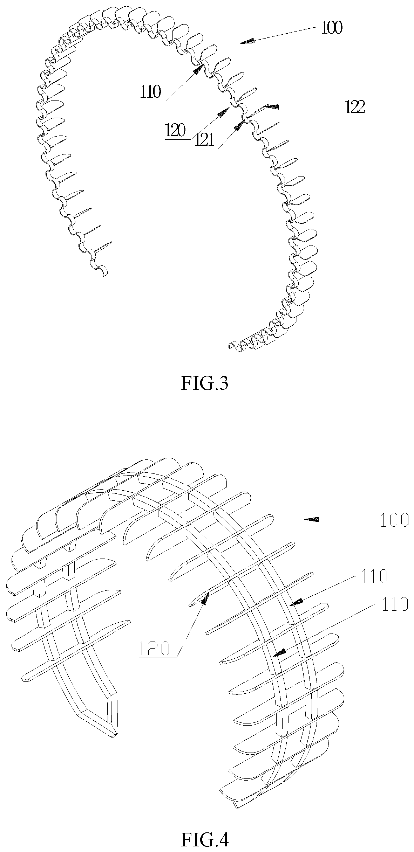

Furthermore, the shape configurations of the hair-entering portion 121 and the maintaining portion 122 shown in the drawings are only examples, and the present disclosure is not limited thereto. For example, in FIG. 3, the first bridging portion 110 and the first hair-entering member 120 are integrally molded structures and constructed in a wave shape, with the wave trough forming the hair-entering portion 121, and the maintaining portion 122 is connected to the hair-entering portion 121 and constructed in a plate shape. It can be appreciated that the maintaining portion 122 may also be constructed in the form of an arc-shaped structure matching the arc shape of the wave trough, as long as it can achieve the function of maintaining the optical path, and a person skilled in the art can realize the function of maintaining the optical path according to hair property. The skilled person may design flexibly according to the hair property, the processing difficulty of the component, the assembly difficulty, etc., and the present disclosure does not make specific limitations in this regard. Therefore, both the first bridging portion 110 and the first hair-entering member 120 may be made of any material capable of transmitting transcranial light, such as a transparent material, such that the external transcranial light can also be directly transmitted to the scalp via the first bridging portion 110, or, the external transcranial light enters, via the first bridging portion 110, the first hair-entering member 120 disposed thereon and is thus transmitted to the scalp. In a preferred embodiment, the first bridging portion 110 and the first hair-entering member 120 are made of the same material and integrated molded.

It will be appreciated that the extending length of the maintaining portion 122 from the first bridging portion 110 is at least a first threshold, so that the optical path formed on the scalp of the user during the first hair-entering member 120 parting the hairs is still maintained by the maintaining portion 122. Particularly, the first threshold may be set in accordance with the relative parameter of the target illumination region of the transcranial light, so that the optical path formed on the scalp during the first hair-entering member 120 travels in the target illumination region is maintained by the maintaining portion 122. For example, when irradiating a region of the head from the forehead to the top, the hair parting assembly 100 may be utilized to part the hairs in the region by traveling from a hair growth edge of the forehead toward the cranial vertex. The extending length of the maintaining portion 122 from the first bridging portion 110 illustrated in FIG. 2 is such that it can at least reach the hair growth edge of the forehead when the first bridging portion 110 is stabilized on the head, that is, it can maintain as much of the optical path formed on the scalp during the first hair-entering member 120 traveling in that region as possible, preventing the hair from returning to obstruct the optical path. In this manner, the first hair-entering member 120 is able to transmit more transcranial light to the scalp.

It will be appreciated that different numbers of first bridging portions 110 may be provided to satisfy the specific needs of different lengths of hairs. The first bridging portions 110 may be provided to be plural, a plurality of first bridging portions 110 are provided in sequence in the lengthwise direction of the first hair-entering member 120, which is particularly suitable for users with short hair. The first hair-entering portion 120 parts the hairs converged on both sides thereof during traveling, after the first hair-entering member 120 has traveled a certain distance, the hairs tending to gradually disperse is again pressed, bunched, and converged by the next first bridging portion 110 (or other circumferential extension portion(s)) disposed sequentially together with the first hair-entering member 120 in a group, so as to avoid obstructing the optical path. Thus, it is possible to converge the short hairs better and prevent it from being disheveled and affecting the optical path. Further, there is a first predetermined distance between the respective first bridging portions 110, and the first predetermined distance is set in accordance with the hair length of the user such that the shorter the hairs of the user are, the shorter the first predetermined distance is. Thus, the convergence of the hairs is controlled by setting the number of the first bridging portions 100 and the first predetermined distance between the respective first bridging portions 110, and a higher optical transmission rate can be realized.

As shown in FIG. 4, the first bridging portions 110 are two, and the two first bridging portions 110 are assembled with the first hair-entering member 120 in sequence in the lengthwise direction of the first hair-entering member 120, and the first predetermined distance between the two first bridging portions 110 is maintained by the first hair-entering member 120. It can be understood that FIG. 4 is only an example, and the present disclosure is not limited thereto, and there is no specific limitation on the number, the shape and structure of the first bridging portions 110, as well as its connection relationship and connection manner with the first hair-entering member 120, etc., and the person skilled in the art may design the first bridging portions 110 and the first hair-entering member 120 in accordance with the length of the user's hairs, head forms and size, etc., and an control the convergence of hairs through the above conception, so as to achieve a better light transmission effect.

In some embodiments, the first hair-entering member 120 may be plural, and the first hair-entering members 120 are bridged by the first bridging portion 110, so that more hairs in the head region can be parted by providing the plurality of first bridging portions 120 to improve the irradiation area of the transcranial light. For example, when it is necessary to irradiate the whole head of the user with the transcranial light, the transcranial light can be transmitted to the scalp of each region by means of the plurality of first hair-entering members 120 correspondingly provided in the respective regions, and a higher light transmission rate can be realized without limiting the relative positional relationship between the first hair-entering members 120 and the external optical member.

It should be noted that the present disclosure does not specifically limit the number of the first hair-entering members 120, and the person skilled in the art may set the number of the first hair-entering members 120, that is, the degree of sparseness of the individual first hair-entering members 120 on the first bridging portion 110, according to the hair property of the user, the target irradiation region of the transcranial light, and so on, it is possible to realize smooth interposion between the hairs of the user and part the hairs, and to transmit more transcranial light to the scalp.

In a preferred embodiment, the first hair-entering member 120 is made of elastic material, and the first hair-entering member 120 made of the elastic material can be well adapted to the head forms and sizes of different users and can be stably placed on the head, and, the elasticity of the first hair-entering member 120 makes it have a certain degree of comfort when it is in contact with the scalp, and does not cause injury to the scalp, and can enhance the use experience.

It will be appreciated that the first hair-entering member 120 is made of a material, through which the light with a wavelength ranging from 600 nm to 1100 nm is transmittable. Research has shown that transcranial light in this wavelength range has good therapeutic effects on cerebral diseases. As an example, at least one transcranial light with a wavelength of at least one of 810 nm, 635 nm, and 1064 nm can be utilized to treat Alzheimer's disease.

It can be understood that the first bridging portion 110 may be made of any material capable of transmitting transcranial light, such as a transparent material, such that the external transcranial light can be transmitted directly to the scalp via the first bridging portion 110, or that the external transcranial light enters the first hair-entering member 120 disposed thereon via the first bridging portion 110, and is thus transmitted to the scalp. In a preferred embodiment, the first bridging portion 110 and the first hair-entering member 120 are made of the same material by means of integrated molding.

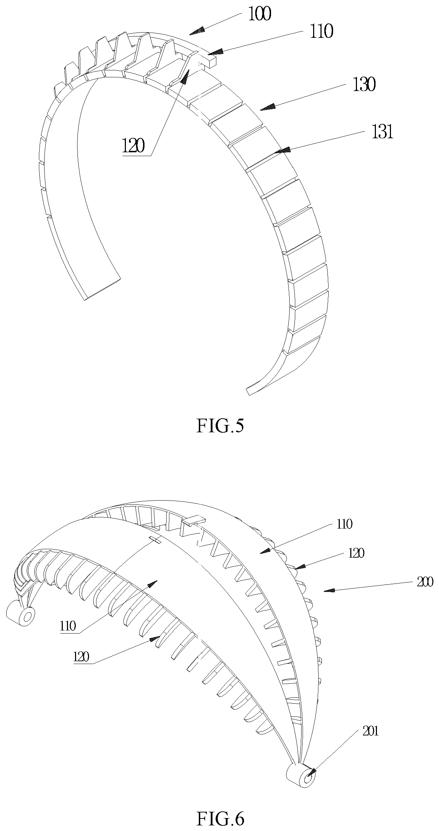

In some embodiments, the hair parting assembly 100 further comprises a first fixing member 130 for fixing to the head and exposing the hairs to be parted (e.g., tightening to the head and gathering up the hairs), and the first hair-entering member 120 is mounted in a detachable manner to the first fixing member 130. As shown in FIG. 5, the first fixing member 130 is constructed in an arc-shaped structure so as to be worn on the head of the user, and is provided with a plurality of mounting holes 131 thereon. The first bridging portion 110 is constructed as a partially arc-shaped structure, the first hair-entering members 120 provided thereon are assembled to the first fixing member 130 in a detachable manner during operation, so that the first hair-entering members 120 can be stabilized on the user's head, avoiding its movement or detachment from affecting the formed optical path. Besides, the hair parting assembly 100 illustrated in FIG. 5 is more flexible, so that an operator can flexibly adjust the mounting position of the first hair-entering members 120 in accordance with the target irradiation area of the transcranial light.

In some embodiments, at least one first hair-entering member 120 may be mounted to the first bridging portion 110 in a detachable manner without the need to introduce additional fixing members. Specifically, for example, a socket may be provided in the first bridging portion 110 to selectively receive and fix the tail portion of the first hair-entering member 120 as needed, so as to enhance the flexibility of the hair parting assembly 100, enabling an operator to flexibly adjust the mounting position of the first hair-entering member 120 in accordance with the target irradiation area of the transcranial light.

According to a second embodiment of the present disclosure, there is provided a hair parting device 200 comprising at least two hair parting assemblies 100 according to various embodiments of the present disclosure. Specifically, the head of the human body can be divided into a plurality of small regions, and a corresponding hair parting assembly 100 can be configured on each of the small regions to achieve a better light transmission effect. In particular, when transcranial light irradiation of the whole brain is required, the hair parting assembly 100 needs to transmit the transcranial light to each region, and the human head has an irregular shape, and configuring the corresponding hair parting assembly 100 on each region of the head can realize a better light transmission effect.

In some embodiments, the at least two hair parting assemblies 100 are pivotally connected with each other at two ends. As shown in FIG. 6, the hair-entering device 200 comprises two hair-entering assemblies 100, two first bridging portions 110 constructed in an arc-shaped structure are pivotally connected with each other at two ends by a rotating shaft 201, and the hair-entering directions of the hair-entering portions 121 of the two first hair-entering members 120 are set to be in opposite directions, so that during operation, an operator can make the hair parting areas of the two hair parting assemblies 110 cover the whole head by pivoting the two bridging portions 110 in two opposite directions from the cranial vertex towards the forehead and the back of the head. Since the two ends of the two first bridging portions 110 are pivotally connected, the two hair parting assemblies 100 can be stably placed on the head during the hair parting process, preventing them from moving and falling off, and affecting the formed optical path. In another embodiment, the hair-entering directions of the hair-entering portions 121 of the two first hair-entering members 120 may also be set in the same direction, and an operator may part the hair along the same direction, which is not specifically limited by the present disclosure.

In some embodiments, the hair parting assemblies 100 can be connected with each other in a detachable manner, which ensures that it can be stably placed on the user's head, while the operator can also flexibly configure the number of hair parting assemblies, hair-parting area, etc. according to the actual needs, so as to make it more personalized.

It can be understood that, in the adjacent hair parting assemblies 100, the hair-entering portion 121 of the first hair-entering member 120 of one hair parting assembly 100 is connected in a detachable manner to the maintaining portion of the first hair-entering member of another hair parting assembly 100, so that the optical path formed by the two hair parting assemblies 100 in the traveling process is coherent, which can avoid that hairs parted by the two hair parting assemblies 100 affect each other and cover the nearby optical path, affecting the light transmission effect.

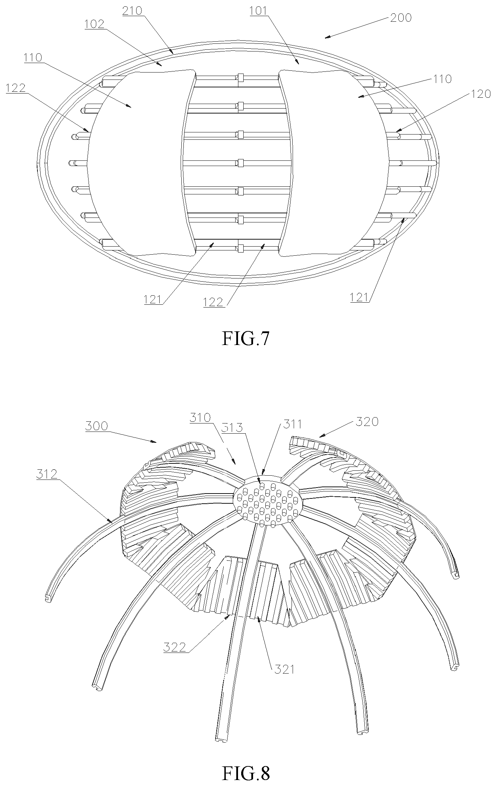

It can be understood that the hair parting assembly 200 further comprises a second fixing member 210 (as shown in FIG. 7) for fixing to the head and exposing the hairs to be parted, and at least one hair parting assembly 100 is connected in a detachable manner to the second fixing member 210. Specifically, the second fixing member 210 is used to stably fix the hair parting assembly 100 to the head, preventing the hair parting assembly 100 from falling off from the head or moving during transcranial light irradiation and affecting the optical path. The second fixing member 210 may be constructed in any shape such as a circle, ellipse, arc shape, etc., and the present disclosure does not make any specific limitations thereon.

In a preferred embodiment, the second fixing member 210 has elasticity and may be constructed as an elastic structure or made of elastic material, so that it can be adapted to users with different head forms and can maintain a certain degree of comfort to enhance the use experience.

It can be understood that at least one hair parting assembly 100 is connected in a detachable manner to the second fixing member 210, so that the hair parting device 200 is flexible and more operable, and the operator can select a suitable hair parting assembly 100 for the user according to the actual demand, or when a plurality of mounting holes are provided on the second fixing member 210, the operator can select the mounting position of the hair parting assembly 100 according to the target irradiation area of the transcranial light.

FIG. 7 illustrates a specific embodiment, which shows an operation state of the hair parting device 200. As shown in FIG. 7, the hair parting device 200 comprises the second fixing member 210, the first hair parting assembly 101, and the second hair parting assembly 102. Under an inoperation state, the three are separate members. During operation state, an operator utilizes the first hair parting assembly 101 to travel from a cranial vertex position toward the back of the head to part the hairs located on the back of the user's head, by assembling the hair-entering portion 121 of the first hair-entering member 120 in the first hair parting assembly 101 to the second fixing member 210, an operator utilizes the second hair parting assembly 102 to travel from a forehead position toward the cranial vertex position to part the hairs located in the forehead, and by means of assembling the hair-entering portion 121 of the first hair-entering member 120 of the second hair parting assembly 102 to the first hair parting assembly 101, it will work as long as the maintaining portion 122 of the first hair-entering member 120 of the second hair parting assembly 102 is placed on the scalp and can be stably maintained on the head without being assembled with the second fixing member 210. Besides, since the hairs on the forehead have been parted by the second hair parting assembly 102 and are converged to the head, the transcranial light can be well transmitted to the scalp. In some embodiments, if the end of the second hair parting assembly 102 away from the first hair parting assembly 101 is also assembled with the second fixing member 210, the comfort of use may be affected instead.

It can be understood that the second fixing member 210 may also be constructed as an arc-shaped structure (not shown in FIG. 7) so as to be easily worn on the head of the user, the first hair parting assembly 101 and the second hair parting assembly 102 are provided on two opposite sides of the second fixing member 210, and the first bridging portion 110 of each hair parting assembly is connected to the second fixing member 210. Thus during operation, the operator may place each hair parting assembly in a cranial vertex position, and achieve the parting of hairs by moving the hair parting assembly to two sides respectively.

It should be noted that FIG. 7 is only an example of the present disclosure illustrating the above-described inventive concept, but the present disclosure is not limited thereto. For example, in some embodiments, the hair parting device may include a plurality of hair parting assemblies, each hair parting assembly corresponds to a different region so as to transmit the transcranial light onto more regions. In addition, considering factors such as structural stability and flexibility, the person skilled in the art may flexibly set the assembly position and assembly relationship between the individual hair parting assemblies and between the hair parting assemblies and the second fixing member 210. For example, the second hair parting assembly 102 may also be connected to the second fixing member 210 in a detachable manner to improve stability, and the first hair parting assembly 101 may be connected to the second fixing member 210 via the first bridging portion 110, or, the hair-entering portion 121 of the first hair-entering member 120 of the second hair parting assembly 102 may also be detachably connected to the first bridging portion 110 of the first hair parting assembly 101, or the like.

According to a third embodiment of the present disclosure, the present disclosure also provides a hair parting assembly 300. As shown in FIG. 8, the hair parting assembly 300 comprises a second bridging portion 310 and at least one second hair-entering member 320 for transmitting transcranial light. The second bridging portion 310 extends at least partially in a circumferential direction and has elasticity so as to be able to be stably worn on a user's head, and each second hair-entering member 320 is provided to the second bridging portion 310 in a slidable manner in the extension direction of the second bridging portion 310, such that hairs can be parted by sliding the second hair-entering member 320 along the extension direction of the second bridging portion 310, and external transcranial light is transmitted to the scalp via the second hair-entering member 320 and the optical path formed by the second hair-entering member 320 in the sliding path.

It can be understood that the present disclosure does not specifically limit the structural shape and production material of the second bridging portion 310 and the structural shape, production material, quantity, etc., of the second hair-entering member 320, which may be the same as or different from the first bridging portion 110 and the first hair-entering member 120, so long as the second bridging portion 310 can be worn stably on the head of the user, and the second hair-entering member 320 can be interposed between the hairs of the user and capable of being used to transmit transcranial light, which is not elaborated here. Thus, the second bridging portion 310 may be made of any material capable of transmitting the transcranial light, such as a transparent material, such that the external transcranial light can also be transmitted directly to the scalp via the second bridging portion 310, or the external transcranial light enters, via the second bridging portion 310, the second hair-entering member 320 provided thereon, and is thus transmitted to the scalp. In a preferred embodiment, the second bridging portion 310 and the second hair-entering member 320 are made of the same material by means of integrated molding.

The above inventive concept is illustrated below using FIG. 8 as an example.

As shown in FIG. 8, the second bridging portion 310 comprises a base portion 311 and a plurality of grasping portions 312 provided around the base portion 311, the second hair-entering members 320 are plural, and the respective second hair-entering members 320 are provided to the grasping portions 312 in a slidable manner in the extension direction of the grasping portions 312. In some embodiments, the plurality of second hair-entering members 320 can surround the second bridging portion 310 in a ring shape, i.e., can cover the entire head of the user in a circumferential direction.

In some embodiments, a body structure 321 of the second hair-entering member 320 tapers from its first side to its second side, and the second side is closer to the root of the grasping portion 312 compared to the first side, i.e., the second side is closer to the cranial vertex position. In this way, it can adapt to the user's head shape and part as much areas of hairs as possible to increase the irradiation area of the transcranial light. Further, a comb teeth portion 322 may be provided on the side of the body structure 321 near the scalp for parting hairs. During operation, the hairs in the path in the extension direction of the grasping portions 312 may be parted by sliding the second hair-entering members 320 downwardly from the cranial vertex position, and a part of the transcranial light is transmitted to the scalp via the optical path maintained by the second hair-entering member 320. Furthermore, a part of the external transcranial light may be directly transmitted to the scalp via the optical path in the traveling path of the second hair-entering members 320.

In some embodiments, as shown in FIG. 8, a light guide member 313 is provided at a side of the base portion 311 close to the scalp of the user. Preferably, the light guide member 313 is constructed in the shape of a light guide pillar, so that the light guide pillars can be interposed into the hairs located at the cranial vertex during operation to contact the scalp of the user, so as to realize the transmission of transcranial light to the position of the cranial vertex.

It can be understood that the structural shape of the comb teeth portion 322, the density of the comb teeth, and the like, are not specifically limited in the present disclosure, and that the person skilled in the art may adopt flexibly designs in accordance with the hair property of the user, the adaptability of the device, and the like, as long as a higher light transmission efficiency can be realized.

It should be noted that FIG. 8 is only an example of the present disclosure illustrating the above inventive concept, but the present disclosure is not limited thereto, for example, the person skilled in the art may flexibly set the structural shape of the second bridging portion 310, the number of grasping portions 312, and the distribution position, and so on, according to the target irradiation area of the transcranial light.

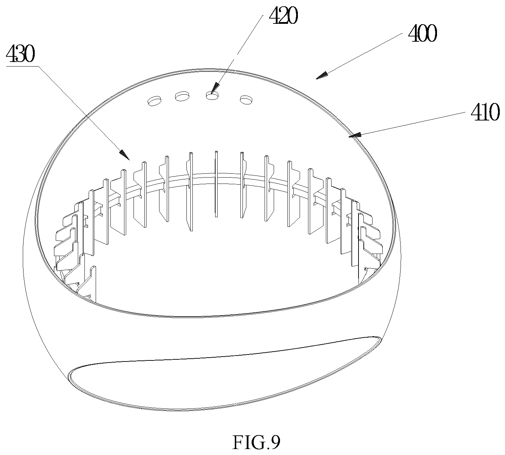

As shown in FIG. 9, the present disclosure also provides a transcranial light regulation apparatus 400. The transcranial light regulation apparatus 400 comprises a carrying member 410 and an optical assembly 420 provided on the carrying member 410 for transmitting transcranial light, and further comprises at least one of the hair parting assembly 430 and the hair parting device (not shown in FIG. 9) as described in any embodiment of the present disclosure.

Specifically, the carrying member 410 may be constructed as any structure capable of carrying the optical assembly 420, such as a helmet, an elastic headgear, and the like. The optical assembly 420 comprises an optical member, which may be any device that can transmit transcranial light, such as a light emitting diode, a laser, a fiber optic, etc. It will be appreciated that the optical members may be plural, and that the plurality of optical members may emit light with different types of parameters. As an example, some of the optical members emit light at a wavelength of 635 nm, and some of the optical members emit light with a wavelength of 810 nm. Particularly, the transcranial light regulation apparatus 400 also comprises a control device (not shown in FIG. 9), wherein the control device can control the light emitting frequency, light emitting pattern, and other operation parameters of each optical member to achieve better disease therapeutic effects. For example, the optical members that can emit different wavelengths emit the transcranial light in a simultaneous or alternating operation mode, or the operation parameters of the optical members are controlled for different diseases. For example, the operation parameters of the optical members with an optical wavelength of 810 nm, pulsed mode, a frequency of 10 Hz, and an optical power density of 50 mW/cm² can be adopted to treat Alzheimer's disease.

Further, the hair parting device or the hair parting assembly 430 is distinct from the optical assembly 420, and the optical assembly 420 transmits the transcranial light outside the hair parting device or the hair parting assembly 430 and transmits (delivers) to the scalp via the hair parting device or the hair parting assembly 430. That is, the hair parting assembly 430 or the hair parting device is not limited by the structure, providing position, and other factors of the optical assembly 420. As shown in FIG. 9, the optical assembly 420 is provided on the carrying member 410, and the hair parting assembly 430, as a separate member, can be used in cooperation with the optical assembly 420, which is not provided on the same member as the optical assembly 420, and the two are not connected. In some embodiments, the placement position of the hair parting assembly 430 on the head may correspond to the target irradiation area of the optical assembly 420, or the mounting position of the hair-entering member on the bridging portion may be adjusted according to the target irradiation area of the optical assembly 420, in order to make the transcranial light regulation apparatus more personalized.

In some embodiments, the hair parting assembly 430 may also be assembled to the carrying member 410 to facilitate the storage or use.

In some embodiments, the transcranial light regulation apparatus 400 further comprises at least one processor (not shown in FIG. 9), which may be configured to control the location and/or direction of the optical assembly 420 with respect to the hair parting assembly 430 or hair parting device, and control the optical assembly 420 to transmit the transcranial light in association with its location and/or direction. Particularly, factors such as the structural shape and size of the hair parting assembly 430 or the hair parting device may affect the transmission rate of the transcranial light, in this case, the transmission rate of the transcranial light may be improved by controlling and adjusting, by the processor, the location and/or direction of the optical assembly 420 with respect to the hair parting assembly 430 or the hair parting device to transmit the transcranial light in association with the same. It should be noted that, in the present disclosure, the location and/or direction of the optical assembly 420 with respect to the hair parting assembly 430 or the hair parting device comprises, but is not limited to, location and/or direction in physical space. For example, in one embodiment, the transcranial light can be focused on a target area by shortening the distance between the optical assembly 420 and the scalp of the user. In another embodiment, the transcranial light irradiated on the scalp can be made to achieve a desired value by changing the parameters of the optical assembly 420, such as the optical power density, frequency, and the like.

In yet another embodiments, when the transcranial light regulation apparatus 400 controls the optical assembly 420 to transmit the transcranial light in association with its location and/or direction, it may specifically comprises controlling the optical assembly 420 to change the strength and irradiation mode of the transmitted transcranial light in association with its location and/or direction, wherein the irradiation mode may include, but is not limited to, the wave length and transmission time period of the transcranial light, etc.

It will be appreciated that the transcranial light regulation apparatus 400 may be used to treat a variety of cerebral diseases, such as Alzheimer's disease of neurodegenerative disorders, depression and autism of psychiatric disorders, cerebral apoplexy of traumatic disorders, and the like, and the present disclosure does not specifically limit this.

The transcranial light regulation apparatus 400 provided in the present disclosure parts the hairs on the scalp of the user by means of the hair parting assembly 430 or the hair parting device, and forms an optical path at the hair-entering member and in its traveling path, so as to realize the transmission of the transcranial light to the scalp. In this manner, the light transmission rate is higher, so as to make the transcranial light regulation apparatus 400 capable of achieving a better therapeutic effect, and the hair parting assembly 430 or the hair parting device is not limited by the structure, arrangement position and other factors of the optical assembly 420, and the structure is simple and the cost is lower.

Furthermore, although exemplary embodiments have been described herein, their scope comprises any and all embodiments having equivalent elements, modifications, omissions, combinations (e. g., schemes crossed by various embodiments), adaptations or changes based on the present disclosure. The elements in the claims will be interpreted broadly based on the language used in the claims, not limited to the examples described in the description or during the implementation of the present application, and the examples will be interpreted as non-exclusive. Therefore, the specification and examples are intended to be considered as examples only, and the true scope are indicated by the following claims

The above description is intended to be illustrative rather than restrictive. For example, the above examples (or one or more of them) may be used in combination with each other. For example, those skilled in the art may use other embodiments when reading the above description. In addition, in the above specific embodiment, various features can be grouped together to simplify the present disclosure. This should not be interpreted as the intention that a disclosed feature that is not claimed is necessary for any claim. On the contrary, the subject matter of the present application may be less than all the features of a particular disclosed embodiment. Thus, the following claims are hereby incorporated into the specific embodiments as examples or embodiments, wherein each claim is independently regarded as a separate embodiment, and it is considered that these embodiments can be combined with each other in various combinations or arrangements. The scope of the present application shall be determined by reference to the full scope of the appended claims.

The above embodiments are only exemplary embodiments of the present disclosure and are not intended to limit the present disclosure. The protection scope of the application is defined by the claims. Those skilled in the art may make various modifications or equivalent substitutions to the present disclosure within the substance and protection scope of the present disclosure, and such modifications or equivalent substitutions shall also be deemed to fall within the protection scope of the application.

## Claims

1. A light guide device for transcranial light, **characterized in that**, comprising:
a first bridging portion (110), which at least partially extends in a circumferential direction in a strip-shape or a band-shape; and
at least one first hair-entering member (120), each first hair-entering member (120) includes a hair-entering portion (121) and a maintaining portion (122), both of which are permeable to the transcranial light and is provided traverse to the first bridging portion (110), such that a lengthwise direction of each first hair-entering member is traverse to the extension direction of the first bridging portion (110), and spaces are provided on both sides of each first hair-entering member (120) in the extension direction of the first bridging portion (110);
wherein, the first bridging portion (110) is configured to travel along the scalp of a user, the first hair-entering member (120) is configured to part the hairs as the first bridging portion (110) travels to expose the scalp in the traveling path to form an optical path, and the maintaining portion (122) is configured to maintain the optical path via which external transcranial light is transmitted to the scalp.

2. The light guide device of claim 1, **characterized in that**, the hair-entering portion (121) is configured to interpose between the hairs of the user, the maintaining portion (122) is light transmittable.

3. The light guide device of claim 1, **characterized in that**, the hair-entering portion (121) and the maintaining portion (122) are distributed on two opposite sides of the first bridging portion (110).

4. The light guide device of claim 1, **characterized in that**, at least a part of the maintaining portion (122) and the hair-entering portion (121) are distributed on the same side of the first bridging portion (110).

5. The light guide device of any one of claims 1-4, **characterized in that**, the bottom of the first bridging portion (110) has a first predetermined height with respect to the bottom of the first hair-entering member (120), the first predetermined height is configured to gather the hair in the spaces on the two sides of the first hair-entering member (120).

6. The light guide device of any one of claims 1-4, **characterized in that**, at least a part of the first hair-entering member (120) has a second predetermined height, which is set associated with the hair property of the user.

7. The light guide device of claim 5, **characterized in that**, at least a part of the first hair-entering member (120) has a second predetermined height, the height difference of the second predetermined height compared to the first predetermined height is set associated with the hair property of the user, including at least one of hair thickness, hair amount, and hair elasticity.

8. The light guide device of any one of claims 1-4, **characterized in that**, the length of the maintaining portion (122) is set in accordance with the target illumination region of the transcranial light.

9. The light guide device of any one of claims 1-4, **characterized in that**, the first hair-entering members (120) are plural, and the first bridging portion (110) is configured to bridge the first hair-entering members (120).

10. The light guide device of any one of claims 1-4, **characterized in that**, the first bridging portion (110) is made of elastic material, or the circumferential extending shape of the first bridging portion (110) provides the elasticity.

11. The light guide device of any one of claims 1-4, **characterized in that**, the light guide device further comprises a first fixing member (130) for tightening to the head and gathering up the hairs, and the first hair-entering member (120) is mounted in a detachable manner to the first fixing member (130).

12. The light guide device of any one of claims 1-4, **characterized in that**, at least one of the first bridging portion (110) and the hair-entering portion (121) is made of a material which can transmit the transcranial light.

13. The light guide device of any one of claims 1-4, **characterized in that**, further comprising at least one circumferential extension portion, which is spaced apart from the first bridging portions (110) in the longitudinal extension direction of the first hair-entering member (120).

14. The light guide device of claim 13, **characterized in that**, there is a first predetermined distance between adjacent portions of the first bridging portion (110) and the circumferential extension portions, the first predetermined distance is set in accordance with the hair length of the user.

15. The light guide device of any one of claims 1-4, **characterized in that**, the first hair-entering member (120) is connected to the first bridging portion (110) in a detachable manner.

16. A light guide device for transcranial light, **characterized in that**, comprising at least two light guide devices of any one of claims 1-15.

17. The light guide device of claim 16, **characterized in that**, in the adjacent light guide devices, the hair-entering portion (121) of the first hair-entering member (120) of one light guide device is connected in a detachable manner to the maintaining portion (122) of the first hair-entering member (120) of another light guide device.

18. A transcranial light regulation apparatus (400), comprising a carrying member (410) and optical assembly (420) provided on the carrying member (410) for transmitting transcranial light, **characterized by** further comprising the light guide device for transcranial light of any one of claims 1-17.

19. The transcranial light regulation apparatus (400) of claim 18, **characterized in that**, the transcranial light regulation apparatus (400) further comprises a control device, the optical assembly (420) comprises an optical member, and depending on type of disease, the control device is configured to control operation parameters of the optical member to emit transcranial light with a wavelength ranging from 600nm to 1100nm, and in a case where the light guide device parts hairs on the scalp corresponding to a target irradiation area to form an optical path, the transcranial light irradiates the scalp via the optical path for treating at least one of neurodegenerative disease, psychiatric disorder, and traumatic disorder.

20. The transcranial light regulation apparatus (400) of claim 18, **characterized in that**, the transcranial light regulation apparatus (400) further comprises a control device , the optical assembly (420) comprises an optical member, and depending on type of disease, the control device is configured to control operation parameters of the optical member to emit transcranial light with a wavelength ranging from 600nm to 1100nm, and in a case where the light guide device parts hairs on the scalp corresponding to a target irradiation area to form an optical path, the transcranial light irradiates the scalp via the optical path for treating Alzheimer's disease.

## Patentansprüche

1. Eine Lichtleitvorrichtung für transkranielles Licht, **dadurch gekennzeichnet, dass** sie umfasst:
einen ersten Überbrückungsabschnitt (110), der sich zumindest teilweise in einer Umfangsrichtung streifenförmig oder bandförmig erstreckt; und
mindestens ein erstes Haareintrittselement (120), wobei jedes erste Haareintrittselement (120) einen Haareintrittsabschnitt (121) und einen Halteabschnitt (122) umfasst, die beide für das transkranielle Licht durchlässig sind und quer zum ersten Überbrückungsabschnitt (110) vorgesehen sind, so dass eine Längsrichtung jedes ersten Haareintrittselements quer zur Erstreckungsrichtung des ersten Überbrückungsabschnitts (110) verläuft und auf beiden Seiten jedes ersten Haareintrittselements (120) in der Erstreckungsrichtung des ersten Überbrückungsabschnitts (110) Räume vorgesehen sind;
wobei der erste Überbrückungsabschnitt (110) dazu eingerichtet ist, entlang der Kopfhaut eines Benutzers zu verlaufen, das erste Haareintrittselement (120) dazu eingerichtet ist, beim Verlaufen des ersten Überbrückungsabschnitts (110) die Haare zu scheiteln, um die Kopfhaut im Verlaufspfad freizulegen und einen optischen Pfad zu bilden, und der Halteabschnitt (122) dazu eingerichtet ist, den optischen Pfad aufrechtzuerhalten, durch den externes transkranielles Licht zur Kopfhaut übertragen wird.

2. Die Lichtleitvorrichtung nach Anspruch 1, **dadurch gekennzeichnet, dass** der Haareintrittsabschnitt (121) dazu eingerichtet ist, zwischen die Haare des Benutzers einzudringen, und der Halteabschnitt (122) lichtdurchlässig ist.

3. Die Lichtleitvorrichtung nach Anspruch 1, **dadurch gekennzeichnet, dass** der Haareintrittsabschnitt (121) und der Halteabschnitt (122) auf zwei gegenüberliegenden Seiten des ersten Überbrückungsabschnitts (110) verteilt sind.

4. Die Lichtleitvorrichtung nach Anspruch 1, **dadurch gekennzeichnet, dass** zumindest ein Teil des Halteabschnitts (122) und des Haareintrittsabschnitts (121) auf derselben Seite des ersten Überbrückungsabschnitts (110) verteilt ist.

5. Die Lichtleitvorrichtung nach einem der Ansprüche 1-4, **dadurch gekennzeichnet, dass** die Unterseite des ersten Überbrückungsabschnitts (110) eine erste vorbestimmte Höhe bezüglich der Unterseite des ersten Haareintrittselements (120) aufweist, wobei die erste vorbestimmte Höhe dazu eingerichtet ist, das Haar in den Räumen auf beiden Seiten des ersten Haareintrittselements (120) zu sammeln.

6. Die Lichtleitvorrichtung nach einem der Ansprüche 1-4, **dadurch gekennzeichnet, dass** zumindest ein Teil des ersten Haareintrittselements (120) eine zweite vorbestimmte Höhe aufweist, die in Zusammenhang mit der Haarausprägung des Benutzers festgelegt ist.

7. Die Lichtleitvorrichtung nach Anspruch 5, **dadurch gekennzeichnet, dass** zumindest ein Teil des ersten Haareintrittselements (120) eine zweite vorbestimmte Höhe aufweist, wobei der Höhenunterschied der zweiten vorbestimmten Höhe im Vergleich zur ersten vorbestimmten Höhe in Zusammenhang mit der Haarausprägung des Benutzers festgelegt ist, einschließlich zumindest einer der folgenden Eigenschaften: Haardicke, Haarmenge und Haarelastizität.

8. Die Lichtleitvorrichtung nach einem der Ansprüche 1-4, **dadurch gekennzeichnet, dass** die Länge des Halteabschnitts (122) in Übereinstimmung mit dem Zielbeleuchtungsbereich des transkraniellen Lichts festgelegt ist.

9. Die Lichtleitvorrichtung nach einem der Ansprüche 1-4, **dadurch gekennzeichnet, dass** die ersten Haareintrittselemente (120) mehrfach vorhanden sind und der erste Überbrückungsabschnitt (110) dazu eingerichtet ist, die ersten Haareintrittselemente (120) zu überbrücken.

10. Die Lichtleitvorrichtung nach einem der Ansprüche 1-4, **dadurch gekennzeichnet, dass** der erste Überbrückungsabschnitt (110) aus elastischem Material besteht oder dass die umfangsseitige Erstreckungsform des ersten Überbrückungsabschnitts (110) die Elastizität bereitstellt.

11. Die Lichtleitvorrichtung nach einem der Ansprüche 1-4, **dadurch gekennzeichnet, dass** die Lichtleitvorrichtung ferner ein erstes Fixierelement (130) umfasst, das zum Festziehen am Kopf und zum Zusammenhalten der Haare dient, und dass das erste Haareintrittselement (120) lösbar am ersten Fixierelement (130) befestigt ist.

12. Die Lichtleitvorrichtung nach einem der Ansprüche 1-4, **dadurch gekennzeichnet, dass** zumindest einer des ersten Überbrückungsabschnitts (110) und des Haareintrittsabschnitts (121) aus einem Material besteht, das das transkranielle Licht übertragen kann.

13. Die Lichtleitvorrichtung nach einem der Ansprüche 1-4, **dadurch gekennzeichnet, dass** sie zumindest einen umfangsseitigen Erstreckungsabschnitt umfasst, der in Längserstreckungsrichtung des ersten Haareintrittselements (120) vom ersten Überbrückungsabschnitt (110) beabstandet ist.

14. Die Lichtleitvorrichtung nach Anspruch 13, **dadurch gekennzeichnet, dass** zwischen benachbarten Abschnitten des ersten Überbrückungsabschnitts (110) und den umfangsseitigen Erstreckungsabschnitten ein erster vorbestimmter Abstand vorhanden ist, wobei der erste vorbestimmte Abstand in Übereinstimmung mit der Haarlänge des Benutzers festgelegt ist.

15. Die Lichtleitvorrichtung nach einem der Ansprüche 1-4, **dadurch gekennzeichnet, dass** das erste Haareintrittselement (120) in lösbarer Weise mit dem ersten Überbrückungsabschnitt (110) verbunden ist.

16. Eine Lichtleitvorrichtung für transkranielles Licht, **dadurch gekennzeichnet, dass** sie zumindest zwei Lichtleitvorrichtungen nach einem der Ansprüche 1-15 umfasst.

17. Die Lichtleitvorrichtung nach Anspruch 16, **dadurch gekennzeichnet, dass** bei benachbarten Lichtleitvorrichtungen der Haareintrittsabschnitt (121) des ersten Haareintrittselements (120) einer Lichtleitvorrichtung in lösbarer Weise mit dem Halteabschnitt (122) des ersten Haareintrittselements (120) einer anderen Lichtleitvorrichtung verbunden ist.

18. Eine transkranielle Lichtreguliervorrichtung (400), umfassend ein Trageelement (410) und eine optische Baugruppe (420), die am Trageelement (410) zum Übertragen von transkraniellem Licht vorgesehen ist, **dadurch gekennzeichnet, dass** die Vorrichtung ferner die Lichtleitvorrichtung für transkranielles Licht nach einem der Ansprüche 1-17 umfasst.

19. Die transkranielle Lichtreguliervorrichtung (400) nach Anspruch 18, **dadurch gekennzeichnet, dass** die transkranielle Lichtreguliervorrichtung (400) ferner eine Steuervorrichtung umfasst, die optische Baugruppe (420) ein optisches Element umfasst und je nach Krankheitstyp die Steuervorrichtung dazu eingerichtet ist, Betriebsparameter des optischen Elements zu steuern, um transkranielles Licht mit einer Wellenlänge von 600 nm bis 1100 nm auszusenden, und dass, wenn die Lichtleitvorrichtung die Haare auf der Kopfhaut in einem Zielbestrahlungsbereich scheitelt, um einen optischen Pfad zu bilden, das transkranielle Licht die Kopfhaut über den optischen Pfad zur Behandlung zumindest einer der folgenden Erkrankungen bestrahlt: neurodegenerative Erkrankung, psychische Störung und traumatische Störung.

20. Die transkranielle Lichtreguliervorrichtung (400) nach Anspruch 18, **dadurch gekennzeichnet, dass** die transkranielle Lichtreguliervorrichtung (400) ferner eine Steuervorrichtung umfasst, die optische Baugruppe (420) ein optisches Element umfasst und je nach Krankheitstyp die Steuervorrichtung dazu eingerichtet ist, Betriebsparameter des optischen Elements zu steuern, um transkranielles Licht mit einer Wellenlänge von 600 nm bis 1100 nm auszusenden, und dass, wenn die Lichtleitvorrichtung die Haare auf der Kopfhaut in einem Zielbestrahlungsbereich scheitelt, um einen optischen Pfad zu bilden, das transkranielle Licht die Kopfhaut über den optischen Pfad zur Behandlung der Alzheimer-Krankheit bestrahlt.

## Revendications

1. Un dispositif de guidage de lumière pour lumière transcrânienne, **caractérisé en ce qu'**il comprend :
une première partie de pontage (110), qui s'étend au moins partiellement dans une direction circonférentielle sous une forme de bande ou de ruban ; et
au moins un premier élément d'entrée de cheveux (120), chaque premier élément d'entrée de cheveux (120) comprenant une partie d'entrée de cheveux (121) et une partie de maintien (122), toutes deux perméables à la lumière transcrânienne et disposées transversalement à la première partie de pontage (110), de sorte qu'une direction longitudinale de chaque premier élément d'entrée de cheveux soit transversale à la direction d'extension de la première partie de pontage (110), et des espaces soient prévus de chaque côté de chaque premier élément d'entrée de cheveux (120) dans la direction d'extension de la première partie de pontage (110) ;
la première partie de pontage (110) étant configurée pour se déplacer le long du cuir chevelu d'un utilisateur, le premier élément d'entrée de cheveux (120) étant configuré pour séparer les cheveux lorsque la première partie de pontage (110) se déplace afin d'exposer le cuir chevelu sur la trajectoire de déplacement pour former un trajet optique, et la partie de maintien (122) étant configurée pour maintenir le trajet optique via lequel la lumière transcrânienne externe est transmise au cuir chevelu.

2. Le dispositif de guidage de lumière selon la revendication 1, **caractérisé en ce que** la partie d'entrée de cheveux (121) est configurée pour s'interposer entre les cheveux de l'utilisateur, et la partie de maintien (122) est transmissive à la lumière.

3. Le dispositif de guidage de lumière selon la revendication 1, **caractérisé en ce que** la partie d'entrée de cheveux (121) et la partie de maintien (122) sont réparties sur deux côtés opposés de la première partie de pontage (110).

4. Le dispositif de guidage de lumière selon la revendication 1, **caractérisé en ce qu'**au moins une partie de la partie de maintien (122) et de la partie d'entrée de cheveux (121) sont réparties sur le même côté de la première partie de pontage (110).

5. Le dispositif de guidage de lumière selon l'une quelconque des revendications 1-4, **caractérisé en ce que** le bas de la première partie de pontage (110) présente une première hauteur prédéterminée par rapport au bas du premier élément d'entrée de cheveux (120), la première hauteur prédéterminée étant configurée pour rassembler les cheveux dans les espaces situés de chaque côté du premier élément d'entrée de cheveux (120).

6. Le dispositif de guidage de lumière selon l'une quelconque des revendications 1-4, **caractérisé en ce qu'**au moins une partie du premier élément d'entrée de cheveux (120) présente une seconde hauteur prédéterminée, laquelle est définie en fonction de la nature des cheveux de l'utilisateur.

7. Le dispositif de guidage de lumière selon la revendication 5, **caractérisé en ce qu'**au moins une partie du premier élément d'entrée de cheveux (120) présente une seconde hauteur prédéterminée, la différence de hauteur de la seconde hauteur prédéterminée par rapport à la première hauteur prédéterminée étant définie en fonction de la nature des cheveux de l'utilisateur, incluant au moins l'une des caractéristiques suivantes-: épaisseur des cheveux, quantité de cheveux et élasticité des cheveux.

8. Le dispositif de guidage de lumière selon l'une quelconque des revendications 1-4, **caractérisé en ce que** la longueur de la partie de maintien (122) est définie conformément à la région d'illumination cible de la lumière transcrânienne.

9. Le dispositif de guidage de lumière selon l'une quelconque des revendications 1-4, **caractérisé en ce que** les premiers éléments d'entrée de cheveux (120) sont multiples, et la première partie de pontage (110) est configurée pour relier les premiers éléments d'entrée de cheveux (120).

10. Le dispositif de guidage de lumière selon l'une quelconque des revendications 1-4, **caractérisé en ce que** la première partie de pontage (110) est réalisée en matériau élastique, ou **en ce que** la forme d'extension circonférentielle de la première partie de pontage (110) fournit l'élasticité.

11. Le dispositif de guidage de lumière selon l'une quelconque des revendications 1-4, **caractérisé en ce que** le dispositif de guidage de lumière comprend en outre un premier élément de fixation (130) destiné à être serré sur la tête et à rassembler les cheveux, et le premier élément d'entrée de cheveux (120) est monté de manière détachable sur le premier élément de fixation (130).

12. Le dispositif de guidage de lumière selon l'une quelconque des revendications 1-4, **caractérisé en ce qu'**au moins l'une de la première partie de pontage (110) et de la partie d'entrée de cheveux (121) est réalisée en un matériau capable de transmettre la lumière transcrânienne.

13. Le dispositif de guidage de lumière selon l'une quelconque des revendications 1-4, **caractérisé en ce qu'**il comprend en outre au moins une partie d'extension circonférentielle, laquelle est espacée de la première partie de pontage (110) dans la direction d'extension longitudinale du premier élément d'entrée de cheveux (120).

14. Le dispositif de guidage de lumière selon la revendication 13, **caractérisé en ce qu'**il existe une première distance prédéterminée entre des parties adjacentes de la première partie de pontage (110) et des parties d'extension circonférentielle, la première distance prédéterminée étant définie en fonction de la longueur des cheveux de l'utilisateur.

15. Le dispositif de guidage de lumière selon l'une quelconque des revendications 1-4, **caractérisé en ce que** le premier élément d'entrée de cheveux (120) est relié de manière détachable à la première partie de pontage (110).

16. Un dispositif de guidage de lumière pour lumière transcrânienne, **caractérisé en ce qu'**il comprend au moins deux dispositifs de guidage de lumière selon l'une quelconque des revendications 1-15.

17. Le dispositif de guidage de lumière selon la revendication 16, **caractérisé en ce que**, dans les dispositifs de guidage de lumière adjacents, la partie d'entrée de cheveux (121) du premier élément d'entrée de cheveux (120) d'un dispositif de guidage de lumière est reliée de manière détachable à la partie de maintien (122) du premier élément d'entrée de cheveux (120) d'un autre dispositif de guidage de lumière.

18. Un appareil de régulation de lumière transcrânienne (400), comprenant un élément porteur (410) et un ensemble optique (420) disposé sur l'élément porteur (410) pour transmettre la lumière transcrânienne, **caractérisé en ce qu'**il comprend en outre le dispositif de guidage de lumière pour lumière transcrânienne selon l'une quelconque des revendications 1-17.

19. L'appareil de régulation de lumière transcrânienne (400) selon la revendication 18, **caractérisé en ce que** l'appareil de régulation de lumière transcrânienne (400) comprend en outre un dispositif de contrôle, l'ensemble optique (420) comprenant un élément optique, et **en ce que**, selon le type de maladie, le dispositif de contrôle est configuré pour contrôler les paramètres de fonctionnement de l'élément optique afin d'émettre une lumière transcrânienne ayant une longueur d'onde comprise entre 600 nm et 1100 nm, et dans le cas où le dispositif de guidage de lumière sépare les cheveux sur le cuir chevelu correspondant à une zone cible d'irradiation pour former un trajet optique, la lumière transcrânienne irradie le cuir chevelu via le trajet optique pour traiter au moins l'une des affections suivantes : maladie neurodégénérative, trouble psychiatrique et trouble traumatique.

20. L'appareil de régulation de lumière transcrânienne (400) selon la revendication 18, **caractérisé en ce que** l'appareil de régulation de lumière transcrânienne (400) comprend en outre un dispositif de contrôle, l'ensemble optique (420) comprenant un élément optique, et **en ce que**, selon le type de maladie, le dispositif de contrôle est configuré pour contrôler les paramètres de fonctionnement de l'élément optique afin d'émettre une lumière transcrânienne ayant une longueur d'onde comprise entre 600 nm et 1100 nm, et dans le cas où le dispositif de guidage de lumière sépare les cheveux sur le cuir chevelu correspondant à une zone cible d'irradiation pour former un trajet optique, la lumière transcrânienne irradie le cuir chevelu via le trajet optique pour traiter la maladie d'Alzheimer.
